(19) **Europäisches Patentamt**

**European Patent Office**

**Office européen des brevets**

(11) **EP 3 418 302 A1**

(12) **EUROPEAN PATENT APPLICATION**

(43) Date of publication:
26.12.2018 Bulletin 2018/52

(51) Int Cl.:
*C07K 16/28* (2006.01)    *A61K 47/00* (2006.01)
*A61K 39/395* (2006.01)

(21) Application number: 17176540.7

(22) Date of filing: 19.06.2017

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **F. Hoffmann-La Roche AG 4070 Basel (CH)**

(72) Inventor: **The designation of the inventor has not yet been filed**

(74) Representative: **Beyermann, Jochen Carl F. Hoffmann-La Roche AG CLP - Patent Department Grenzacherstrasse 124 4070 Basel (CH)**

(54) **ADMINISTRATION ROUTES FOR IMMUNE AGONISTS**

(57)    Disclosed herein is a new use of liquid pharmaceutical preparations comprising immune agonistic antibodies, resulting in improved tolerability/adverse event profile while at least maintaining the same level of biological activity of said immune agonist.

EP 3 418 302 A1

**Description**

The present invention relates to the field of parenteral antibody formulations, in particular subcutaneous (sc) pharmaceutical preparations of antibodies, or antibody fragments, acting as immune agonists.

## BACKGROUND OF THE INVENTION

Immunomodulatory antibodies offer an treatment approach and might be used to directly potentiate anti-tumor immune responses or as adjuvants for anti-cancer vaccines (Melero, I., et al. Nat Rev Cancer 7, 2007, 95-106). Immune agonists, especially agonistic anti-CD40 antibodies constitute one of the most effective classes of these reagents. CD40 is a cell-surface member of the tumor necrosis factor superfamily expressed on antigen presenting cells (APCs) such as dendritic cells, B cells and macrophages. Preclinical studies with anti-CD40 agonists suggest that triggering CD40 with crosslinking antibodies on antigen presenting cells (APCs) can substitute for CD4 T cell help, normally provided via CD40 ligand, and facilitate the activation as well as expansion of CD8 effector T cells (Li, F. et al, Science 333, 2011, 1030-1034). In addition CD40-activated macrophages may also exert direct tumoricidal functions (Beatty, G. L., et al. Science 331, 2011, 1612-1616; Vonderheide, R. H., et al., Oncoimmunology 2, 2013, e23033). CD40 agonists are disclosed in WO 2003/040170.

Conventional, intravenous (iv) administration of immune agonists is often associated with unfavorable adverse event or tolerability profiles which may result in less than optimal therapeutic efficacy of said immune agonist or even interruption of treatment (see e.g. Vonderheide R. H., et al., Clin Cancer Res 19(5), 2013, 1035-1043). Subcutaneous (sc) administration of antibodies may be used to overcome some of the effects seen with intravenous antibody formulations. Some s.c. formulations of antibodies are known (Lundin J., et al., Blood 100 (3), 2002, 2001, 768-773; Davies A. et al., Lancet Oncol 15(3), 2014, 343-352) with some commercial products being available. However, the majority of these formulations comprise antagonistic antibodies, and often specific excipients, such as e.g. hyaluronidase are needed to achieve the desired effect. There remains a need to search for new, alternative pharmaceutical formulations applicable for sc administration of immune agonists.

Pharmaceutical compositions of the specific CD40 agonist disclosed herein are for example disclosed in WO2003/040170.

## BRIEF SUMMARY OF THE INVENTION

The present invention comprises the use of a liquid pharmaceutical preparation comprising an immune agonist for the treatment of a patient suffering from cancer or an infectious disease, wherein said liquid pharmaceutical preparation is administered subcutaneously and wherein said subcutaneous administration provides for improved tolerability and at least equal efficacy compared to other parenteral administration routes of that same preparation.

The invention further comprises the use as described above wherein the immune agonist is an antibody or an antigen-binding portion thereof that specifically binds to and activates human CD40.

The invention further comprises a liquid pharmaceutical preparation comprising an immune agonist for use in treating cancer or an infectious disease, characterized in that it is administered subcutaneously.

The invention further comprises an injection device comprising the liquid pharmaceutical formulation as defined herein.

The invention further comprises a kit comprising vials of the liquid pharmaceutical preparation, optionally together with an injection device for subcutaneous administration to a patient in accordance with the present invention.

The invention further comprises a method of treating a patient suffering from an infectious disease or a proliferative disease, such as cancer, said method comprises subcutaneous administration of a liquid pharmaceutical preparation comprising an immune modulator, in particular an anti-CD40 antibody.

## BRIEF DESCRIPTION OF THE FIGURES

**Fig. 1:** Comparison of PK data ($C_{max}$) of an iv - and an sc administration in accordance with the present invention in monkeys.

**Fig. 2**: Maintenance of at least the same biological activity by subcutaneous administration compared to intravenous administration, as demonstrated by

(a) B cell activation subsequent to subcutaneous administration, compared to intravenous administration;

**(b)** T cell activation subsequent to subcutaneous administration, compared to intravenous administration.

## SEQUENCE LISTINGS

SEQ ID NO 1: human CD40
SEQ ID NO 2: light chain variable domain (VL) of a CD40 antibody according to the present invention.
SEQ ID NO 3: heavy chain variable domain (VH) of a CD40 antibody according to the present invention.

## DETAILED DESCRIPTION OF THE INVENTION

Monoclonal antibodies with agonistic function against activating molecules on immune cells such as CD3 (Topp M.S., et al, Lancet Oncol. 16(1), 2015, 57-66), CD28 (Suntharalingam, G., et al, N Engl J Med. 355(10), 2006, 1018-28) may lead to cytokine release after intravenous administration which may be dose limiting.

CD40, a member of the tumor necrosis factor receptor (TNFR) superfamily, is a critical regulator of the anti tumor immune response via its expression on antigen presenting cells (APCs) that include B lymphocytes, dendritic cells (DCs), and monocytes (see e.g. Grewal IS et al, Ann Rev Immunol, 1998;16:111-35; Van Kooten C et al, J Leukoc. Biol , 2000;67:2-17; or O'Sullivan B et al, Crit Rev Immunol. 2003;23(1 2):83-107). CD40 stimulated DCs up regulate antigen processing and presentation pathways and migrate to lymph nodes to activate naive T cells. Agonist CD40 antibodies were shown to substitute the function of CD4+ lymphocytes resulting in cytotoxic T lymphocyte (CTL) expansion able to clear established lymphoma in murine models (see e.g. Sotomayor EM et al, Nature Medicine, 1999;5(7):780-7; Gladue RP et al, Cancer Immunol Immunother, 2011;60(7):1009-17). CD40 agonists trigger immune stimulation by activating host APCs, which then drive T cell responses directed against the tumor (see e.g. Vonderheide RH, Clin CancerRes, 2007;13:1083-8).

The inventors of the present invention have found that a ready to use pharmaceutical preparation, which has previously been used for i.v. administration, can be used without modification for s.c. administration. The use of said preparation subcutaneously leads to improved tolerability and/or adverse event profile in patients while at the same time the full biological activity of the immune agonists is at least maintained or even improved (NCT02665416, NCT02304393). Maintenance of the full biological activity of said immune agonists is for example demonstrated by the ability of activating immune cells, which leads proliferation (determined based on Ki67 expression in these cells, e.g. CD8 T cells) and/or migration of these cells (e.g. CD20 expressing B cells) from the peripheral blood to other compartements in the body. Moreover, the subcutaneous use in accordance with the present invention leads to higher doses of said immune agonists being administered within one treatment cycle, therefore resulting in higher efficacy per treatment cycle with improved tolerability.

Therefore, in one embodiment, the present invention comprises a liquid pharmaceutical preparation comprising an immune agonist and a pharmaceutically acceptable carrier for use in the subcutaneous (sc) administration to a patient suffering from cancer, or an infectious disease, wherein said use is characterized by improving the adverse event profile compared to other parenteral administration routes, especialy intravenous (iv) injections, of that same immune agonist in the same pharmaceutically acceptable carrier. The subcutaneous administration of said liquid pharmaceutical preparation comprising said immune agonist maintains at least the same, or substantially the same, biological activity as if said liquid pharmaceutical preparation comprising said immune agonist would be injected intravenously. In certain embodiments of the present invention, the biological activity of said immune agonist is even improved following an sc administration in accordance with the present invention when compared to an iv administration of the same pharmaceutical preparation.

An "immune agonist", as used herein combines with a receptor on a cell and initiates a reaction or activity that is similar to or the same as that initiated by a natural ligand of the receptor.

A "CD40 agonist", as used herein, induces any or all of, but not limited to, the following responses: B cell proliferation and/or differentiation; upregulation of intercellular adhesion via such molecules as ICAM- 1, E-selectin, VC AM, and the like; secretion of pro-inflammatory cytokines such as IL-1, IL-6, IL-8, IL-12, TNF, and the like; signal transduction through the CD40 receptor by such pathways as TRAF {e.g., TRAF2 and/or TRAF3), MAP kinases such as NIK (NF-kB inducing kinase), I-kappa B kinases (IKK /.beta.), transcription factor NF-kB, Ras and the MEK/ERK pathway, the PI3K AKT pathway, the P38 MAPK pathway, and the like; transduction of an anti-apoptotic signal by such molecules as XIAP, mcl-1, bcl-x, and the like; B and/or T cell memory generation; B cell antibody production; B cell isotype switching, up-regulation of cell-surface expression of MHC Class II and CD80/86, and the like.

By agonist activity is intended an agonist activity (read out by parameters such as cell proliferation or induction of costimulatory molecules such as CD80 or CD86 on CD40 expressing cells such as B lymphocytes) that is at least 3 standard deviations higher than the mean effect induced by an irrelevant isotype control antibody using the same readout.

The "CD40 agonist" (or "CD40 antibody") as used herein includes any moiety that agonizes the CD40/CD40L interaction. Typically these moieties will be agonistic CD40 antibodies or agonistic CD40L polypeptides. These antibodies include by way of example human antibodies, chimeric antibodies, humanized antibodies, bispecific antibodies, scFvs, and antibody fragments that specifically agonize the CD40/CD40L binding interaction. In one embodiment the agonistic CD40 antibody will comprise a chimeric, fully human or humanized CD40 antibody. In another preferred embodiment the agonistic CD40 antibody will comprise a fully human CD40 antibody.

In another embodiment, the immune agonist as mentioned above is a CD40 agonist or a fragment of a CD40 agonist. The human CD40 antigen, as targeted by the CD40 agonist according to the present invention, is a 50 kDa cell surface glycoprotein which belongs to the Tumor Necrosis Factor Receptor (TNF-R) family (Stamenkovic et al., EMBO J. 8:1403-10 (1989)). It is also known as "Tumor necrosis factor receptor superfamily member 5". Alternative designations include B-cell surface antigen 40, Bp50, CD40L receptor, CDw40, CDW40, MGC9013, p50 or TNFRSF5. It is for example registered under UniProt Entry No. P25942. In one embodiment human CD40 antigen has the sequence according to SEQ ID NO: 1 (see Table 1).

**Table 1:** Protein sequence of human CD40 antigen

| SEQ ID NO:1 | MVRLPLQCVLWGCLLTAVHPEPPTACREKQYLINSQC CSLCQPGQKLVSDCTEFTETECLPCGESEFLDTWNRE THCHQHKYCDPNLGLRVQQKGTSETDTICTCEEGWH CTSEACESCVLHRSCSPGFGVKQIATGVSDTICEPCPV GFFSNVSSAFEKCHPWTSCETKDLVVQQAGTNKTDV VCGPQDRLRALVVIPIIFGILFAILLVLVFIKKVAKKPT NKAPHPKQEPQEINFPDDLPGSNTAAPVQETLHGCQP VTQEDGKESR ISVQERQ |
|---|---|

In one embodiment human CD40 has the sequence according to SEQ ID NO: 1. As used herein, "binding to human CD40" or "specifically binding to human CD40" or "which binds to human CD40" or "anti-CD40 antibody" refers to an antibody specifically binding to the human CD40 antigen with a binding affinity of $K_D$-value of $1.0 \times 10^{-8}$ mol/l or less, in one embodiment of a $K_D$-value of $1.0 \times 10^{-9}$ mol/l or less. The binding affinity is determined with a standard binding assay, such as surface plasmon resonance technique (BIAcore®, GE-Healthcare Uppsala, Sweden). Thus an "antibody binding to and activating human CD40" as used herein refers to an antibody specifically binding to the human CD40 antigen with a binding affinity of KD $1.0 \times 10^{-8}$ mol/l or less (in one embodiment $1.0 \times 10^{-8}$ mol/l - $1.0 \times 10^{-13}$ mol/l), in on embodiment of a KD $1.0 \times 10^{-9}$ mol/l or less (in one embodiment $1.0 \times 10^{-9}$ mol/l - $1.0 \times 10^{-13}$ mol/l). In another embodiment, the anti-CD40 antibody according to the present invention binds to human CD40 with a $K_D$ of $4 \times 10^{-10}$ M or less.

In one embodiment of the present invention, the antibody which binds to human CD40 is an agonist ("CD40 agonist").

In one embodiment, said CD40 antibody is a fully human antibody of the IgG2 subclass. In yet another embodiment said antibody is any of the anti-CD40 antibodies as specifically disclosed in WO2003/040170. In still another embodiment the CD40 agonist according to the present invention is selected from the group of antibodies designated 3.1.1, 7.1.2, 10.8.3, 15. 1.1, 21.4.1, 21.2.1, 22.1.1, 23.5.1, 23.25.1, 23.29.1 and 24.2.1 according to WO2003/040170. Hybridomas secreting those antibodies have been deposited in accordance with the Budapest Treaty. Deposit Numbers can be found in para [0250] of WO2003/040170. In another embodiment, the CD40 antibody according to the present invention comprises the heavy chain - and light chain variable domain amino acid sequences of antibody 21.4.1 (ATCC Deposit No. PTA-3605). In yet another embodiment, the CD40 antibody according to the present invention consists of the heavy chain - and light chain amino acid sequences of antibody 21.4.1 (ATCC Deposit No. PTA-3605).

In one embodiment, the human, agonistic anti-CD40 antibody according to the present invention comprises a light chain variable domain of amino acid SEQ ID NO: 2 and a heavy chain variable domain of amino acid SEQ ID NO: 3 (Table 2).

**Table 2:** Amino acid sequences of the light (VL) - and heavy (VH) chain variable domain of a CD40 agonist according to the present invention.

| VL (Amino Acid Sequence) SEQ ID NO: 2 | DIQMTQSPSSVSASVGDRVTITCRASQGIYSWLAWYQQKP GKAPNLLIYTASTLQSGVPSRFSGSGSGTDFTLTISSLQPED FATYYCQQ ANIFPLTFGGGTKVEIK |
|---|---|
| VH (Amino Acid Sequence) SEQ ID NO: 3 | QVQLVQSGAEVKKPGASVKVSCKASGYTFTGYYMHWV RQAPGQGLEWMGWINPDSGGTNYAQKFQGRVTMTRDTS ISTAYMELNRLRSDDTAVYYCARDQPLGYCTNGVCSYFD YWGQGT LVTVSS |

In another embodiment, the CD40 agonist is a fully human antibody of the IgG2 subclass which binds to human CD40 with a $K_D$ of 4 x 10$^{-10}$ M or less; or

an antibody comprising a VL of SEQ ID NO:3 and a VH of SEQ ID NO:4; or

an antibody comprising the heavy chain - and light chain variable domain amino acid sequences of antibody 21.4.1 (ATCC Deposit No. PTA-3605); or

an antibody comprising the heavy chain - and light chain amino acid sequences of antibody 21.4.1 (ATCC Deposit No. PTA-3605); and

the antibody in B) is any of the PD-L1 antibodies mentioned in a) to p) herein before.

The term "improved tolerability" as used herein means less adverse events in a patient compared to other parenteral administration routs of the same liquid pharmaceutical preparation such as, for example, intravenous administration. The term "adverse event" or "adverse event profile" means direct or indirect effects caused by agonism of CD40 on cells, typically but not exclusively manifested via release of cytokines by CD40 expressing immune cells. This profile includes clinical symptoms (e.g. chills, fever, hypotension, etc.) as well changes in serum parameters used to monitor organ function such as liver function tests and coagulation parameters.

The term "subcutaneous administration (sc)" as used herein means administration of the CD40 agonist via injection(s) into the subcutaneous tissue, with or without addition of components to facilitate resorption (e.g. hyaluronidase). SC administration includes the option of splitting the dose and administering to multiple anatomical sites on the same dosing day, and also includes both single and repeated dosing (3-4 weeks apart).

"Liquid pharmaceutical preparations" as used herein are prepared by mixing said immune agonist having the desired degree of purity with one or more optional "pharmaceutically acceptable carriers" (Remington's Pharmaceutical Sciences 16th edition, Osol, A. Ed. (1980)), in the form of aqueous solutions or lyophilized formulations. "Pharmaceutically acceptable carriers", as used herein, are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

The formulations to be used for s.c. administration are generally sterile. Sterility may be readily accomplished, e.g., by filtration through sterile filtration membranes.

In one embodiment, the present invention provides as an immune agonist the CD40 antibody 21.4.1 (ATCC Deposit No. PTA-3605) as disclosed in WO2003/040170 prepared in a concentration of 10 mg/ml in a buffer solution consisting of: 20 mM sodium acetate, 140 mM sodium chloride, 0.02% Polysorbate 80, pH 5.5. The liquid formulation is provided in 2 mL vials, thus containing the CD40 antibody in an amount of 20 mg/vial. This formulation is also disclosed in Example 1 herein.

Actual dosage levels of the immune agonists, in particular CD40 agonists, in the pharmaceutical compositions of the present invention may be varied so as to obtain an amount of the active ingredient which is effective to achieve the desired therapeutic response for a particular patient, composition, and mode of administration, without being toxic to the patient (effective amount). The selected dosage level will depend upon a variety of pharmacokinetic factors including the activity of the particular immune agonists of the present invention employed, or the age, sex, weight, condition, general health and prior medical history of the patient being treated, and like factors well known in the medical arts. In one embodiment, the dose of the CD40 agonist in accordance with the present invention is from about 1 to 100 mg/ml, or from about 10 to 80 mg/ml, or from about 10 to 40 mg/ml, or from about 10 to 30 mg/ml, or from about 5 to 55 mg/ml. In another embodiment, the dose of the CD40 agonist in the accordance with the present invention is about 5 mg/ml, or about 10 mg/ml, or about 15 mg/ml, or about 20 mg/ml, or about 25 mg/ml, or about 30 mg/ml, or about 35 mg/ml, or about 40 mg/ml, or about 45 mg/ml, or about 50 mg/ml, or about 55 mg/ml.

The pH of the compositions according to the present inventions can vary between 5.0 and 6.0. Any physiological acceptable buffer to obtain such pH can be used. In one embodiment the buffer is selected from sodium succinate or sodium acetate, with or without sodium chloride. In another embodiment, the selected buffer is present in an amount from about 10 to 30 mM, especially at about 20 mM, and the pH is about 5.5.

The compositions in accordance of the present invention may contain surfactants. In one embodiment the surfactant is a nonionic surfactant. In another embodiment, the surfactant is selected from the groups of Poloxamers or Polysorbates. In another embodiment the surfactant is Poloxamer-188, or Polysorbate-20, or Polysorbate 80. In accordance with the present invention, the surfactant is present in a concentration of about 0.02 to 0.1 % (w/v). In one embodiment, the surfactant is present in a concentration of 0.02, 0.03, 0.04, 0.05, 0.06, 0.07, 0.08, 0.09 or 0.1 % (w/v).

In yet another embodiment of the present invention, the compositions in accordance with the present invention may optionally contain a cryoprotectant - and/or an antioxidant agent. In one embodiment, the cryoprotectant is a sugar, especiall sucrose and the antioxidant is methionine. The cryoprotectant is present in an amount from about 180-300 nM, or at about 240 nM. The antioxidant is present in an amount from about 0 to 20 mM, or from about 0-10 mM, or at about 10 mM.

The term "cancer" as used herein preferably means a solid tumor such as, for example, lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma, lymphoma, lymphocytic leukemia, including refractory versions of any of the above cancers, or a combination of one or more of the above cancers. In one preferred embodiment such cancer is a breast cancer, colorectal cancer, melanoma, head and neck cancer, lung cancer or prostate cancer. In one embodiment such cancer is a solid tumor selected from breast cancer, lung cancer, colon cancer, ovarian cancer, melanoma cancer, bladder cancer, renal cancer, kidney cancer, liver cancer, head and neck cancer, colorectal cancer, pancreatic cancer, gastric carcinoma cancer, esophageal cancer, mesothelioma or prostate cancer. In another embodiment such cacer is a hematological tumor such as for example, leukemia (such as AML, CLL), lymphoma, myelomas. In still another embodiment the cancer is breast cancer, lung cancer, colon cancer, colorectal cancer, pancreatic cancer, gastric cancer or prostate cancer.

The term "infectious diseases", as used herein, means disorders caused by organisms such as bacteria, viruses, fungi or parasites. Specific examples of infectious diseases comprise, without limitation, Hepatitis B Virus (HBV), Hepatitis C Virus (HCV) and/or Human Immunodeficiency Virus (HIV).

In one embodiment the s.c. administration in accordance with the present invention is for use in the prevention or treatment of metastasis.

In one embodiment the s.c. administration in accordance with the present invention is for use in treating or delaying progression of an immune related disease such as tumor immunity.

In one embodiment the s.c. administration in accordance with the present invention is for use in stimulating an immune response or function, such as T cell activity.

The term "variable domain" (light chain variable domain VL, heavy chain variable domain VH) as used herein denotes each of the pair of light and heavy chain domains which are involved directly in binding the antibody to the antigen. The variable light and heavy chain domains have the same general structure and each domain comprises four framework (FR) regions whose sequences are widely conserved, connected by three "hypervariable regions" (or complementary determining regions, CDRs). The framework regions adopt a beta-sheet conformation and the CDRs may form loops connecting the beta-sheet structure. The CDRs in each chain are held in their three-dimensional structure by the framework regions and form together with the CDRs from the other chain the antigen binding site. The antibody's heavy and light chain CDR3 regions play a particularly important role in the binding specificity/affinity of the antibodies according to the invention and therefore provide a further object of the invention.

The term "antigen-binding portion of an antibody" when used herein refer to the amino acid residues of an antibody which are responsible for antigen-binding. The antigen-binding portion of an antibody comprises amino acid residues from the "complementary determining regions" or "CDRs". "Framework" or "FR" regions are those variable domain regions other than the hypervariable region residues as herein defined. Therefore, the light and heavy chain variable domains of an antibody comprise from N- to C-terminus the domains FR1, CDR1, FR2, CDR2, FR3, CDR3, and FR4. Especially, CDR3 of the heavy chain is the region which contributes most to antigen binding and defines the antibody's properties. CDR and FR regions are determined according to the standard definition of Kabat et al., Sequences of Proteins of Immunological Interest, 5th ed., Public Health Service, National Institutes of Health, Bethesda, MD (1991) and/or those residues from a "hypervariable loop".

In one embodiment of the present invention, the "antigen-binding portion" of an antibody that specifically binds to and activates human CD40 comprises CDR1, CDR2 and CDR3 of the heavy- and light chain variable domains of antibody 21.4.1 (ATCC Deposit No. PTA-3605).

The terms "nucleic acid" or "nucleic acid molecule", as used herein, are intended to include DNA molecules and RNA molecules. A nucleic acid molecule may be single-stranded or double-stranded, but preferably is double-stranded DNA.

The term "amino acid" as used within this application denotes the group of naturally occurring carboxy alpha-amino acids comprising alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

The "Fc part" of an antibody is not involved directly in binding of an antibody to an antigen, but exhibit various effector functions. A "Fc part of an antibody" is a term well known to the skilled artisan and defined on the basis of papain cleavage of antibodies. Depending on the amino acid sequence of the constant region of their heavy chains, antibodies or immunoglobulins are divided in the classes: IgA, IgD, IgE, IgG and IgM, and several of these may be further divided into subclasses (isotypes), e.g. IgG1, IgG2, IgG3, and IgG4, IgA1 and IgA2. According to the heavy chain constant regions the different classes of immunoglobulins are called $\alpha$, $\delta$ $\epsilon$, $\gamma$, and $\mu$, respectively. The Fc part of an antibody is directly involved in ADCC (antibody-dependent cell-mediated cytotoxicity) and CDC (complement-dependent cytotoxicity) based on complement activation, C1q binding and Fc receptor binding. Complement activation (CDC) is initiated by binding of complement factor C1q to the Fc part of most IgG antibody subclasses. While the influence of an antibody on the complement system is dependent on certain conditions, binding to C1q is caused by defined binding sites in the Fc part. Such binding sites are known in the state of the art and described e.g. by Boackle, R.J., et al., Nature 282 (1979) 742-743; Lukas, T.J., et al., J. Immunol. 127 (1981) 2555-2560; Brunhouse, R., and Cebra, J.J., Mol. Immunol. 16 (1979) 907-917; Burton, D.R., et al., Nature 288 (1980) 338-344; Thommesen, J.E., et al., Mol. Immunol. 37 (2000) 995-1004; Idusogie, E.E., et al., J. Immunol.164 (2000) 4178-4184; Hezareh, M., et al., J. Virology 75 (2001) 12161-12168; Morgan, A., et al., Immunology 86 (1995) 319-324; EP 0 307 434. Such binding sites are e.g. L234, L235, D270, N297, E318, K320, K322, P331 and P329 (numbering according to EU index of Kabat, E.A., see below). Antibodies of subclass IgG1, IgG2 and IgG3 usually show complement activation and C1q and C3 binding, whereas IgG4 do not activate the complement system and do not bind C1q and C3.

In one embodiment the immune agonists, preferably CD40 agonists, according to the present invention are monoclonal antibodies. In another embodiment the immune agonists, preferably CD40 agonists, according to the present invention are of human IgG class (i.e. of IgG1, or IgG2, or IgG3 or IgG4 subclass).

In one embodiment the immune agonist, preferably CD40 agonists, described herein is characterized in that the constant chains are of human origin. Such constant chains are well known in the state of the art and e.g. described by Kabat, E.A., (see e.g. Johnson, G. and Wu, T.T., Nucleic Acids Res. 28 (2000) 214-218).

The immune agonist, preferably CD40 agonist, described herein are preferably produced by recombinant means. Such methods are widely known in the state of the art and comprise protein expression in prokaryotic and eukaryotic cells with subsequent isolation of the antibody polypeptide and usually purification to a pharmaceutically acceptable purity. For the protein expression nucleic acids encoding light and heavy chains or fragments thereof are inserted into expression vectors by standard methods. Expression is performed in appropriate prokaryotic or eukaryotic host cells, such as CHO cells, NS0 cells, SP2/0 cells, HEK293 cells, COS cells, yeast, or E. coli cells, and the antibody is recovered from the cells (from the supernatant or after cells lysis).

Recombinant production of antibodies is well-known in the state of the art and described, for example, in the review articles of Makrides, S.C., Protein Expr. Purif. 17 (1999) 183-202; Geisse, S., et al., Protein Expr. Purif. 8 (1996) 271-282; Kaufman, R.J., Mol. Biotechnol. 16 (2000) 151-161; Werner, R.G., Drug Res. 48 (1998) 870-880.

The antibodies may be present in whole cells, in a cell lysate, or in a partially purified, or substantially pure form. Purification is performed in order to eliminate other cellular components or other contaminants, e.g. other cellular nucleic acids or proteins, by standard techniques, including alkaline/SDS treatment, CsCl banding, column chromatography, agarose gel electrophoresis, and others well known in the art. See Ausubel, F., et al., ed. Current Protocols in Molecular Biology, Greene Publishing and Wiley Interscience, New York (1987).

Expression in NS0 cells is described by, e.g., Barnes, L.M., et al., Cytotechnology 32 (2000) 109-123; Barnes, L.M., et al., Biotech. Bioeng. 73 (2001) 261-270. Transient expression is described by, e.g., Durocher, Y., et al., Nucl. Acids. Res. 30 (2002) E9. Cloning of variable domains is described by Orlandi, R., et al., Proc. Natl. Acad. Sci. USA 86 (1989) 3833-3837; Carter, P., et al., Proc. Natl. Acad. Sci. USA 89 (1992) 4285-4289; Norderhaug, L., et al., J. Immunol. Methods 204 (1997) 77-87. A preferred transient expression system (HEK 293) is described by Schlaeger, E.-J. and Christensen, K., in Cytotechnology 30 (1999) 71-83, and by Schlaeger, E.-J., in J. Immunol. Methods 194 (1996) 191-199.

The heavy and light chain variable domains according to the invention are combined with sequences of promoter, translation initiation, constant region, 3' untranslated region, polyadenylation, and transcription termination to form expression vector constructs. The heavy and light chain expression constructs can be combined into a single vector, co-transfected, serially transfected, or separately transfected into host cells which are then fused to form a single host cell expressing both chains.

The control sequences that are suitable for prokaryotes, for example, include a promoter, optionally an operator sequence, and a ribosome binding site. Eukaryotic cells are known to utilize promoters, enhancers and polyadenylation signals.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading frame. However, enhancers do not have to be contiguous. Linking is accomplished by ligation at convenient restriction sites. If such sites do not exist, the synthetic oligonucleotide adaptors or linkers are used in accordance with conventional practice.

The monoclonal antibodies are suitably separated from the culture medium by conventional immunoglobulin purification procedures such as, for example, protein A-Sepharose, hydroxylapatite chromatography, gel electrophoresis, dialysis, or affinity chromatography. DNA and RNA encoding the monoclonal antibodies are readily isolated and sequenced using conventional procedures. The hybridoma cells can serve as a source of such DNA and RNA. Once isolated, the DNA may be inserted into expression vectors, which are then transfected into host cells such as HEK 293 cells, CHO cells, or myeloma cells that do not otherwise produce immunoglobulin protein, to obtain the synthesis of recombinant monoclonal antibodies in the host cells.

As used herein, the expressions "cell", "cell line", and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Variant progeny that have the same function or biological activity as screened for in the originally transformed cell are included.

Methods for producing the specific CD40 agonistic antibody used in accordance with the present invention are also disclosed in WO2003/040170.

The invention further comprises a method for the treatment of a patient, having cancer, characterized by subcutaneously administering to the patient a therapeutically effective amount of a liquid pharmaceutical preparation comprising an immune agonist, preferably a CD40 agonist, according to the present invention.

The invention further comprises a kit comprising one or more vials containing any of the liquid formulations disclosed herein and an injection device for subcutaneous administration of the formulation to a patient. Within this embodiment the liquid formulation according to Example 1 is preferred.

The invention can thus be summarized by the following more specific embodiments, wherein all variables and terms have the ranges and/or definitions given herein before:

1. A liquid pharmaceutical preparation comprising an immune agonist for use in the treatment of cancer or an infectious disease, wherein said liquid pharmaceutical preparation is administered subcutaneously.

2. The liquid pharmaceutical preparation for use according to embodiment 1, characterized in that it comprises an aqueous solution comprising:

(a) from about 1-100 mg/ml of said immune agonist;

(b) from about 10-30 mM of at least one buffering agent providing a pH of 5 to 6; and

(c) about 0.01 to 0.1 % (w/v) of a nonionic surfactant.

3. The liquid pharmaceutical preparation for use according to embodiments 1 or 2, characterized in that it comprises an aqueous solution comprising:

(a) from about 10-80 mg/ml of said immune agonist;

(b) about 20 mM of at least one buffering agent providing a pH of about 5.5; and

(c) about 0.02 % (w/v) of a nonionic surfactant.

4. The liquid pharmaceutical preparation for use according to any one of embodiments 1 to 3, further comprisingfrom about 180-300 mM sucrose and from about 0-20 mM methionine.

5. The liquid pharmaceutical preparation for use according to embodiment 4, further comprising about 240 mM Sucrose and from about 0-10 mM Methionine.

6. The liquid pharmaceutical preparation for use according to any one of embodiments 2 to 5, wherein said buffering agent is an acetate or succinate buffer, for example sodium acetate or sodium succinate or a combination thereof, optionally further comprising about 140 mM sodium chloride.

7. The liquid pharmaceutical preparation for use according to any one of embodiments 2 to 6, wherein said non-ionic surfactant is Poloxamer 188 or Polysorbate 20 or Polysorbate 80.

8. The liquid pharmaceutical preparation for use according to any one of embodiments 1 to 7, wherein the immune agonist is an agonistic CD40 antibody.

9. The liquid pharmaceutical preparation for use according to any one of embodiments 1 to 8, characterized in that it is an aqueous solution containing about 10 mg/ml of an agonistic anti CD40 antibody; about 20 mM sodium succinate and about 0.02 % (w/v) of a surfactant selected from Polysorbate 20, Polysorbate 80 or Poloxamer 188 at a pH of about 5.5.

10. The liquid pharmaceutical preparation for use according to any one of embodiments 1 to 9, wherein the immune agonist is an agonistic anti CD40 antibody comprising a light chain variable domain (VL) of SEQ NO: 2 and heavy chain variable domain (VH) of SEQ NO: 3.

11. The liquid pharmaceutical preparation for use according to embodiment 10, wherein the agonistic anti CD40 antibody is the antibody with the ATCC Deposit No. PTA-3605.

12. The liquid pharmaceutical preparation for use according to any one of embodiments 1 to 11, characterized in that said use comprises the treatment of cancer, specifically solid tumors, more specifically adenocarcinoma of the colon and rectum, non-small cell lung cancer, squamous cell carcinoma of the head and neck.

13. The liquid pharmaceutical preparation for use according to any one of embodiments 1 to 11, characterized in that said use comprises the treatment of infectious diseases, especially Hepatitis B Virus (HBV), Hepatitis C Virus (HCV) and/or Human Immunodeficiency Virus (HIV).

14. An injection device for administering the liquid pharmaceutical preparation for the use according to any one of embodiments 1 to 13.

15. A kit comprising one or more vials containing the liquid formulation for use according to any one of embodiments 1 to 13 and an injection device according to embodiment 14 for subcutaneous administration of said formulation to a patient.

16. The use of a liquid pharmaceutical preparation according to any one of embodiments 1 to 11 for the treatment of a patient suffering from cancer or an infectious disease, characterized in that said liquid pharmaceutical preparation is administered subcutaneously.

17. The use of a liquid pharmaceutical preparation according to any one of embodiments 1 to 11 for the manufacture of a medicament for the treatment of cancer or an infectious disease, characterized in that said liquid pharmaceutical preparation is administered subcutaneously.

**EXAMPLES**

**Example 1**

The following example illustrates the preparation of a liquid pharmaceutical preparation in accordance with the present invention. In this example, the immune agonist is the CD40 antibody 21.4.1 (ATCC Deposit No. PTA-3605) as disclosed in WO2003/040170. The following preparations steps were applied under sterile conditions:

1. Preparing a buffer solution (pH 5.5) containing:

- 20 mM sodium acetate,

- 140 mM sodium chloride

2. Concentrating and buffer-exchanging the CD40 antibody into buffer in step 1, using tangential flow filtration.

3. Conditioning the buffer-exchanged product with Polysorbate-80 stock solution to achieve 0.02% (w/v) Polysorbate-80 and a final product concentration of 10 mg/ml.

**Example 2:**

The following example illustrates the assessment of pharmacokinetic (PK) data such as the maximum plasma concentration ($C_{max}$) of the CD40 agonist preparation according to Example 1 in *Cynomolgus* monkey following a single intravenous (iv) and subcutaneous (sc) administration:

2.1 *Cynomolgus* monkeys were dosed once by intravenous or subcutaneous route at a dose level of 0.1, 1.0 and 5.0 mg/kg. Samples for systemic exposure were taken from all animals at predose and scheduled times through 58 days post-dosing:

- IV Administration: predose, 0.08, 0.5, 1, 2, 4, 6, 10, 24 hours post dose and then on Days 3, 4, 5, 7, 9, 16, 22, 29, 43, and 58.

- SC Administration: predose, 0.5, 1, 2, 4, 6, 10, 24 hours post dose and then on Days 3, 4, 5, 7, 9, 16, 22, 29, 43, and 58.

Blood samples for pharmacokinetic evaluations were allowed to clot in tubes for serum preparation for 60 min. at room temperature. The clot were spun down by centrifugation (at least 10 min., 1200g, +4 °C) and stored in a freezer at -80°C pending shipment. The concentration of the CD40 agonistic antibody in cynomolgus monkey serum was determined using a qualified electrochemiluminescence immunoassay (ECLIA) method based on the ELECSYS® 2010/e411 immunoanalyzer platform (Roche Diagnostics, Germany). Samples, prediluted with assay buffer, were incubated with capture and detection molecules for 9 min at 37 °C. Biotinylated mAb<H-Fab(kappa)>M-IgG-Bi was used as capture molecule and a ruthenium(II)tris (bipyridyl)$3^{2+}$[Ru(bpy)$3^{2+}$]-labeled mAb<H-Fc-pan>M-R10Z8E9-F(ab')$_2$-BP-Ru mouse monoclonal antibody was used for detection. Streptavidin-coated magnetic microparticles were added and incubated for additional 9 min at 37 °C to allow complex formation due to biotin-streptavidin interactions. Complexes were magnetically captured on an electrode and a chemiluminescent signal generated using the coreactant tripropylamine (TPA) was measured by a photomultiplier detector. All study samples, and positive or negative control samples were analyzed in replicates and calibrated against the corresponding dosing solution material prepared from the CD40 agonistic antibody.

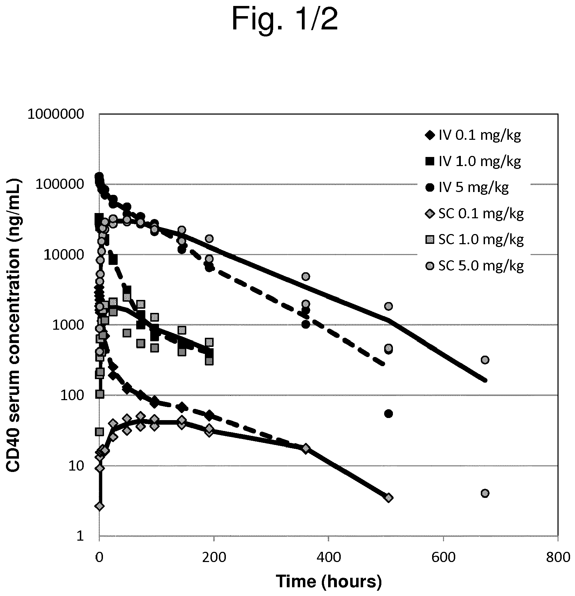

2.2 The s.c. administration resulted in substantially lower $C_{max}$ values due to prolonged absorption with the sc route (see **Fig. 1**). The non-linear bioavailability was apparent showing a bioavailability for sc between 27%-49% in the low (0.1 mg/kg) and mid (1.0 mg/kg) dose group but 100% in the high (5.0 mg/kg) dose. The non-linear bioavailability suggests a saturable first pass effect in the sc tissue and/or the draining lymphatic system.

Thus, the sc administration allows to administer higher CD40 doses than iv and thereby allows to achieve an efficacious dose without inducing Injection Related Reactions (IRRs). This is because iv administration results in very high $C_{max}$ which in turns induces high cytokine release. sc administration, in turn, results in a flatter PK curve, lower $C_{max}$, and no IRR.

### Example 3:

This example demonstrates the improved tolerability/adverse event profile of the subcutaneous (sc) dosing compared to intravenous (iv) injection as observed in clinical trials (Table 3).

**Table 3:** Reported Averse Events (subcutaneous vs intravenous dosing)

| Reported Adverse Event Term | IV Administration (n=29)[1] All causalities [related] | | | | SC Administration (n=36)[2] Cycle 1- all causalities [related] | | | | |
|---|---|---|---|---|---|---|---|---|---|
| Cytokine Release Syndrome (CRS) | 16 (55%), [16 (55%)] | | | | 0(0%) | | | | |
| 1. Chills<br>2. Rigors<br>3. Fever<br>4. Nausea<br>5. Vomiting<br>6. Muscle ache<br>7. Back pain | 1. 4 (13.8%) [4(13.8%)]<br>2. reported as a symptom of CRS<br>3. reported as a symptom of CRS<br>4. 10 (30.5%) [8 (27.6%)]<br>5. 3 (10.3%) [3 (10.3%)]<br>6. 4 (13.8%) [2 (6.9%)]<br>7. 3 (10.3) [2 (6.9%)] | | | | 1. 2 (5.6%) [1(2.8%)]<br>2. -<br>3. 10 (27.8%) [5 (13.9%)]<br>4. 6 (16.7%) [1 (2.8%)]<br>5. 3 (8.3%) [1 (2.8%)]<br>6. 1 (2.8%) [1 (2.8%)]<br>7. - | | | | |
| Aspartate Aminotransferase (AST) increase | **Total 17 (58.6%)** | Gr1 10 | Gr2 5 | Gr3 2 | Gr4 - | **Total 19 (52.8%)** | Gr1 16 | Gr2 3 | Gr3 - | Gr4 - |
| Alanine Aminotransferase (ALT) increase | **Total 11 (37.9%)** | Gr1 7 | Gr2 3 | Gr3 1 | Gr4 - | **Total 13 (36.1%)** | Gr1 12 | Gr2 1 | Gr3 - | Gr4 - |
| Total Bilirubin increase | **Total 5 (17.2%)** | Gr1 2 | Gr2 3 | Gr3 - | Gr4 - | **Total 4 (11.1%)** | Gr1 4 | Gr2 - | Gr3 - | Gr4 - |

[1]weight based dosing 0.01-0.3mg/kg, https://www.ncbi.nlm.nih.gov/pubmed/17327609
[2]flat dosing 1-24mg (equivalent to doses used in IV study), https://clinicaltrials.gov/ct2/show/NCT02665416

### Example 4:

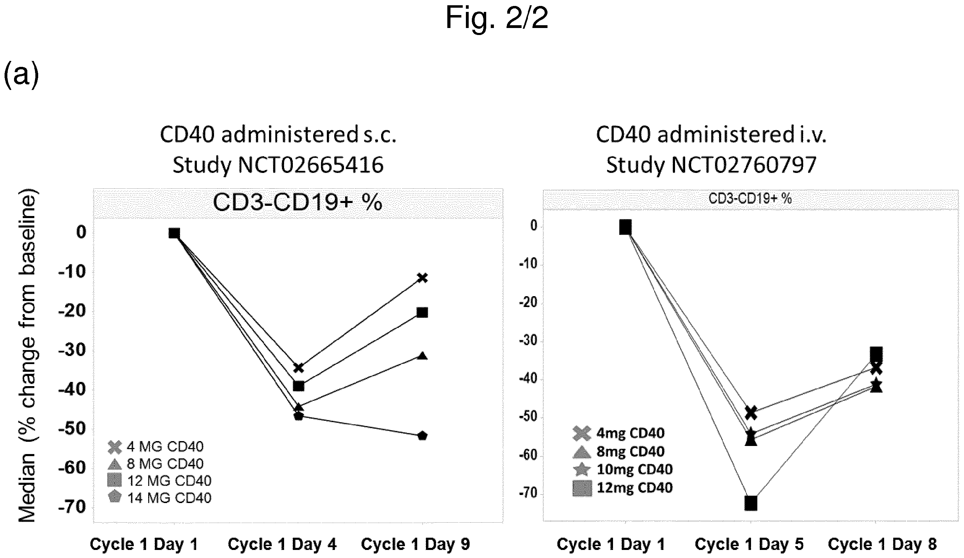

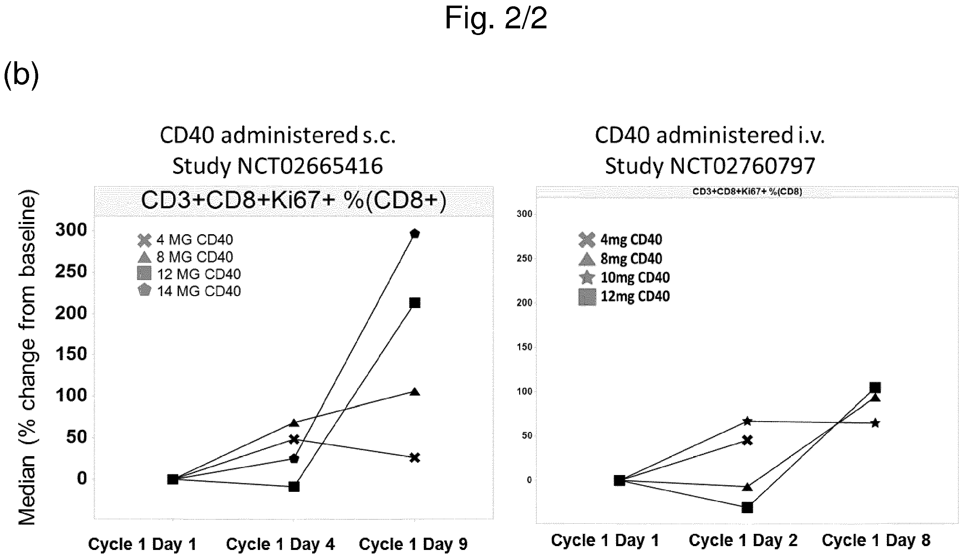

This example demonstrates that the biological activity of the CD40 agonist is at least maintained, or even improved, when switching the same composition from the i.v. to s.c. administration route. Pharmacodynamic comparison suggests similar or stronger Pharmacodynamic after sc than after iv in relevant T-cell markers **(see Fig. 2).**

4.1 Patients

Whole blood samples were collected from patients enrolled in the CD40 studies NCT02665416 and NCT02760797 at baseline and on-study day 4 and day 9 from baseline. For CD3+, CD8+ T cell analysis, whole blood samples were drawn into 2 mL Cyto-Chex Blood Collection Tubes (Streck Cat # 341697-1). For proliferation, CD3+, CD8+, Ki67+ T cell analysis, the whole blood samples were drawn into 2 mL sodium heparin tubes.

4.2 Composition and dosage regimen

The composition used in this Example is as obtained according to Example 1.

4.3 Flow cytometric analysis

At the indicated analysis day, samples were collected from patient that received 4, 8, 10, 12 or 14 mg CD40 injection on day 1 (i.v.) or 2 (s.c.) of the cycle. The frequencies of Cd19+ cells among lymphocytes and that of CD3+, CD8+ T cells, and proliferating CD3+, CD8+, Ki67+ T cells lymphocytes were determined by two different panels using a BD FACSCanto II (8 colour, 3 laser; Becton Dickinson, Franklin Lakes, NJ, USA).

The following anti-human antibodies (Abs) were used to identify the CD19 B cell population and the CD3+, CD8+ T cells population: anti-CD45 PerCP-Cy5.5 (Clone 2D1 cat# 332784), anti-CD3-FITC (Clone SK7 Cat# 345764), and anti-CD8 APC (Clone SK1 Cat# 345775) The following anti-human Abs were used in a different panel to identify the proliferating CD3+, CD8+, Ki67+ T cells population: anti-CD45_V500 (Clone HI30 cat#560777), CD3_APC (Clone UCHT1 cat#555335) CD8_PE (Clone HIT8a cat#555635), CD19 BV421 (Clone HIB19 Cat# 562440) and anti-Ki67_AF488 (Clone B56 cat#558616) . All of these antibodies were provided by BD Biosciences (San Jose, CA, USA). To obtain the absolute cell count, BD TruCOUNT tubes cat#340334 from BD Biosciences were used.The specific cell populations were analyzed by the following gating strategy: from the leukocyte population, CD45+ lymphocytes were identified, which were then gated for CD3+ T cells. Following this step, CD4+ and CD8+ T cell populations were identified through quadrant gating and CD8+ were gated for Ki67+. Percent of CD19+ cells was calculated using the single platform method described bellow among the CD3- cell subset identified within the lymphocytes subset.

4.4 Results

The absolute cell counts for each reportable parameter using a single platform method were calculated by applying the following calculation:

$$\textit{Absolute Cell count} = (\textit{Number of captured cell events/Number of Trucount bead events}) \times (\textit{Trucount absolute number/sample volume (}\mu l))$$

wherein the terms have have the following meanings:

- **Trucount bead events;** Number of events acquired in the bead region as absolute count
- **Trucount absolute number**; This value is found on the TruCOUNT Absolute Count tube foil pouch label and might vary lot to lot.
- **% of captured events (%lymphs);** % of captured events in the lymphocyte gate, of the reportable analyte intended to calculate absolute counts
- **lymphocyte:leukocyte ratio; and** Ratio of lymphocyte to leucocyte is calculated by dividing the lymphocyte event count by the total leucocyte count (leucocyte events and CD 14+ events)
- **WBC: W**hite **B**lood cell **C**ount, from hematology analyzer.

While the dual platform absolute cell counts were calculated by applying the following calculation:

$$\textit{Absolute cell count} = \%\textit{ of captured events (\%lymphs)} \times \textit{lymphocyte:leukocyte ratio} \times \textit{WBC}$$

For proliferating CD3+, CD8+, Ki67+ T cellsabsolute (Cells/$\mu$l) values were calculated indirectly using this dual platform method. Lymphocyte gating was determined by forward scatter (cell size) and side scatter (cell granularity). and FACSDiva TM software (BD Biosciences) was used to analyze data.

Subcutaneous and intravenous CD40 administration induce similar dose-dependent B cell activation as measured by the reduction in the percentage of peripheral B cells on days 4/5 and 8/9 compared to pretreatment values (**Fig. 2a**).

Subcutaneous and intravenous CD40 administration induce T cell activation, which is dose-dependent in sc on Cycle 1 Day 9, as measured by the increase in the percentage of proliferating peripheral CD8+ T cells on days 2/5 and 8/9 compared to pretreatment values (**Fig. 2b**).

### Example 5

This example provides further liquid pharmaceutical preparations which can be prepared and used in accordance with the present invention. Similar to the method disclosed in Example 1, the following liquid pharmaceutical preparations can be prepared in accordance with the present invention, under sterile conditions. The immune agonist is as in Example 1, and can be present in an amout from 1-100 mg/ml.

1. Preparing a buffer solution (pH 5 to 6) containing:

- 10-30 mM sodium succinate, and optionally

- 140 mM sodium chloride

2. Concentrating and buffer-exchanging the CD40 antibody into buffer in step 1, using tangential flow filtration;

3. Conditioning the buffer-exchanged product with 180-300 mM sucrose, 0.02-0.1 % (w/v) Poloxamer-188 or Polysorbate-20, and 0-20 mM methionine to achieve a final product concentration of 1-100 mg/ml (see Table 4).

**Table 4:** Specific CD40 s.c. compositions

| Ex.-No. | CD40 agonist [mg/ml] | Buffer | Sucrose [mM] | Surfactant; [% w/v] | Methionine [mM] |
|---|---|---|---|---|---|
| 5a | 10 | Sodium succinate, 20 mM, (pH 5.5) | 240 | Polysorbate-20; 0.02 | 0 |
| 5b | 10 | Sodium succinate, 20 mM, (pH 5.5) | 240 | Polysorbate-20; 0.02 | 10 |
| 5c | 10 | Sodium succinate, 20 mM, (pH 5.5) | 240 | Poloxamer-188; 0.02 | 0 |
| 5d | 10 | Sodium succinate, 20 mM, (pH 5.5) | 240 | Poloxamer-188; 0.02 | 10 |

### Claims

1. A liquid pharmaceutical preparation comprising an immune agonist for use in the treatment of cancer or an infectious disease, wherein said liquid pharmaceutical preparation is administered subcutaneously.

2. The liquid pharmaceutical preparation for use according to claim 1, **characterized in that** it comprises an aqueous solution comprising:

   (a) from about 1-100 mg/ml of said immune agonist;
   (b) from about 10-30 mM of at least one buffering agent providing a pH of 5 to 6; and
   (c) about 0.01 to 0.1 % (w/v) of a nonionic surfactant.

3. The liquid pharmaceutical preparation for use according to claims 1 or 2, **characterized in that** it comprises an aqueous solution comprising:

   (a) from about 10-80 mg/ml of said immune agonist;
   (b) about 20 mM of at least one buffering agent providing a pH of about 5.5; and
   (c) about 0.02 % (w/v) of a nonionic surfactant.

4. The liquid pharmaceutical preparation for use according to any one of claims 1 to 3, further comprisingfrom about 180-300 mM sucrose and from about 0-20 mM methionine.

**5.** The liquid pharmaceutical preparation for use according to claim 4, further comprising about 240 mM Sucrose and from about 0-10 mM Methionine.

**6.** The liquid pharmaceutical preparation for use according to any one of claims 2 to 5, wherein said buffering agent is an acetate or succinate buffer, for example sodium acetate or sodium succinate or a combination thereof, optionally further comprising about 140 mM sodium chloride.

**7.** The liquid pharmaceutical preparation for use according to any one of claims 2 to 6, wherein said non-ionic surfactant is Poloxamer 188 or Polysorbate 20 or Polysorbate 80.

**8.** The liquid pharmaceutical preparation for use according to any one of claims 1 to 7, wherein the immune agonist is an agonistic CD40 antibody.

**9.** The liquid pharmaceutical preparation for use according to any one of claims 1 to 8, **characterized in that** it is an aqueous solution containing about 10 mg/ml of an agonistic anti CD40 antibody; about 20 mM sodium succinate and about 0.02 % (w/v) of a surfactant selected from Polysorbate 20, Polysorbate 80 or Poloxamer 188 at a pH of about 5.5.

**10.** The liquid pharmaceutical preparation for use according to any one of claims 1 to 9, wherein the immune agonist is an agonistic anti CD40 antibody comprising a light chain variable domain (VL) of SEQ NO: 2 and heavy chain variable domain (VH) of SEQ NO: 3.

**11.** The liquid pharmaceutical preparation for use according to claim 10, wherein the agonistic anti CD40 antibody is the antibody with the ATCC Deposit No. PTA-3605.

**12.** The liquid pharmaceutical preparation for use according to any one of claims 1 to 11, **characterized in that** said use is the treatment of cancer, specifically solid tumors, more specifically adenocarcinoma of the colon and rectum, non-small cell lung cancer, squamous cell carcinoma of the head and neck.

**13.** The liquid pharmaceutical preparation for use according to any one of claims 1 to 11, **characterized in that** said use is the treatment of infectious diseases, especially Hepatitis B Virus (HBV), Hepatitis C Virus (HCV) and/or Human Immunodeficiency Virus (HIV).

**14.** An injection device for administering the liquid pharmaceutical preparation for the use according to any one of claims 1 to 13.

**15.** A kit comprising one or more vials containing the liquid formulation for use according to any one claims 1 to 13 and an injection device according to claim 14 for subcutaneous administration of said formulation to a patient.

**16.** The use of a liquid pharmaceutical preparation according to any one of claims 1 to 11 for the treatment of a patient suffering from cancer or an infectious disease, **characterized in that** said liquid pharmaceutical preparation is administered subcutaneously.

**17.** The use of a liquid pharmaceutical preparation according to any one of claims 1 to 11 for the manufacture of a medicament for the treatment of cancer or an infectious disease, **characterized in that** said liquid pharmaceutical preparation is administered subcutaneously.

Fig. 1/2

(a)

CD40 administered s.c.
Study NCT02665416

CD3-CD19+ %

Median (% change from baseline)

❌ 4 MG CD40
▲ 8 MG CD40
■ 12 MG CD40
⬟ 14 MG CD40

Cycle 1 Day 1    Cycle 1 Day 4    Cycle 1 Day 9

CD40 administered i.v.
Study NCT02760797

CD3-CD19+ %

❌ 4mg CD40
▲ 8mg CD40
★ 10mg CD40
■ 12mg CD40

Cycle 1 Day 1    Cycle 1 Day 5    Cycle 1 Day 8

EP 3 418 302 A1

Fig. 2/2

(b)

CD40 administered s.c.
Study NCT02665416

CD3+CD8+Ki67+ %(CD8+)

Median (% change from baseline)

Legend:
- 4 MG CD40
- 8 MG CD40
- 12 MG CD40
- 14 MG CD40

Cycle 1 Day 1    Cycle 1 Day 4    Cycle 1 Day 9

CD40 administered i.v.
Study NCT02760797

CD3+CD8+Ki67+ %(CD8)

Legend:
- 4mg CD40
- 8mg CD40
- 10mg CD40
- 12mg CD40

Cycle 1 Day 1    Cycle 1 Day 2    Cycle 1 Day 8

EP 3 418 302 A1

Europäisches
Patentamt

European
Patent Office

Office européen
des brevets

## EUROPEAN SEARCH REPORT

Application Number

EP 17 17 6540

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MIERLO VAN G J D ET AL: "CD40 STIMULATION LEADS TO EFFECTIVE THERAPY OF CD40- TUMORS THROUGH INDUCTION OF STRONG SYSTEMIC CYTOTOXIC T LYMPHOCYTE IMMUNITY", PROCEEDINGS NATIONAL ACADEMY OF SCIENCES, NATIONAL ACADEMY OF SCIENCES, US, vol. 99, no. 8, 16 April 2002 (2002-04-16) , pages 5561-5566, XP008046904, ISSN: 0027-8424, DOI: 10.1073/PNAS.082107699 * page 5562, third paragraph; page 5564, paragraph bridging left- and right-hand columns; page 5564, right-hand column, lines 6-8 from the bottom; page 5565, right-hand column, lines 14-17 from the bottom * ----- | 1-17 | INV. C07K16/28 A61K47/00 A61K39/395 |
| X | WO 2010/024676 A1 (ACADEMISCH ZIEKENHUIS LEIDEN H [NL]; HERBERT-FRANSEN MARIEKE FERNAN [N) 4 March 2010 (2010-03-04) * whole document, especially Example 2; Figure 2; claims 1-3 * ----- | 1-17 | |
| X | US 2005/136055 A1 (GLADUE RONALD P [US] ET AL) 23 June 2005 (2005-06-23) * whole document, especially paragraph [0024]; Examples 4-8; SEQ ID NOs 5, 7 * ----- | 1-17 | TECHNICAL FIELDS SEARCHED (IPC) C07K A61K |

The present search report has been drawn up for all claims

| Place of search | Date of completion of the search | Examiner |
|---|---|---|
| The Hague | 16 November 2017 | Luyten, Kattie |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

                                                       
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 17 17 6540

This annex lists the patent family members relating to the patent documents cited in the above-mentioned European search report.
The members are as contained in the European Patent Office EDP file on
The European Patent Office is in no way liable for these particulars which are merely given for the purpose of information.

16-11-2017

| Patent document cited in search report | Publication date | Patent family member(s) | Publication date |
|---|---|---|---|
| WO 2010024676 A1 | 04-03-2010 | AU 2009286247 A1 | 04-03-2010 |
| | | CA 2735421 A1 | 04-03-2010 |
| | | CN 102170908 A | 31-08-2011 |
| | | EP 2323690 A1 | 25-05-2011 |
| | | JP 2012501323 A | 19-01-2012 |
| | | JP 2014224150 A | 04-12-2014 |
| | | US 2011311525 A1 | 22-12-2011 |
| | | WO 2010024676 A1 | 04-03-2010 |
| US 2005136055 A1 | 23-06-2005 | AU 2004308749 A1 | 14-07-2005 |
| | | BR PI0418029 A | 17-04-2007 |
| | | CA 2549652 A1 | 14-07-2005 |
| | | CA 2704300 A1 | 14-07-2005 |
| | | CN 1897971 A | 17-01-2007 |
| | | CN 102552905 A | 11-07-2012 |
| | | DK 2218461 T3 | 17-05-2016 |
| | | EP 1706143 A1 | 04-10-2006 |
| | | EP 2218461 A1 | 18-08-2010 |
| | | EP 3081933 A1 | 19-10-2016 |
| | | ES 2580002 T3 | 18-08-2016 |
| | | HU E027717 T2 | 28-10-2016 |
| | | IL 175540 A | 31-10-2011 |
| | | JP 5634321 B2 | 03-12-2014 |
| | | JP 2007515469 A | 14-06-2007 |
| | | JP 2011184446 A | 22-09-2011 |
| | | JP 2014205692 A | 30-10-2014 |
| | | KR 20070012626 A | 26-01-2007 |
| | | KR 20080023766 A | 14-03-2008 |
| | | NZ 547162 A | 26-03-2010 |
| | | NZ 583179 A | 30-06-2011 |
| | | PL 2218461 T3 | 30-09-2016 |
| | | RU 2355421 C2 | 20-05-2009 |
| | | TW I359671 B | 11-03-2012 |
| | | US 2005136055 A1 | 23-06-2005 |
| | | US 2009311254 A1 | 17-12-2009 |
| | | US 2011104182 A1 | 05-05-2011 |
| | | US 2012263732 A1 | 18-10-2012 |
| | | WO 2005063289 A1 | 14-07-2005 |
| | | ZA 200603804 B | 26-09-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2003040170 A
- US 20050260186 A
- US 20060104968 A
- US 6267958 B
- US 6171586 B
- WO 2006044908 A
- EP 0307434 A

### Non-patent literature cited in the description

- **MELERO, I. et al.** *Nat Rev Cancer,* 2007, vol. 7, 95-106
- **LI, F. et al.** *Science,* 2011, vol. 333, 1030-1034
- **BEATTY, G. L. et al.** *Science,* 2011, vol. 331, 1612-1616
- **VONDERHEIDE, R. H. et al.** *Oncoimmunology,* 2013, vol. 2, e23033
- **VONDERHEIDE R. H. et al.** *Clin Cancer Res,* 2013, vol. 19 (5), 1035-1043
- **LUNDIN J. et al.** *Blood,* 2001, vol. 100 (3), 768-773
- **DAVIES A. et al.** *Lancet Oncol,* 2014, vol. 15 (3), 343-352
- **TOPP M.S. et al.** *Lancet Oncol.,* 2015, vol. 16 (1), 57-66
- **SUNTHARALINGAM, G. et al.** *N Engl J Med.,* 2006, vol. 355 (10), 1018-28
- **GREWAL IS et al.** *Ann Rev Immunol,* 1998, vol. 16, 111-35
- **VAN KOOTEN C et al.** *J Leukoc. Biol,* 2000, vol. 67, 2-17
- **O'SULLIVAN B et al.** *Crit Rev Immunol.,* 2003, vol. 23 (1 2), 83-107
- **SOTOMAYOR EM et al.** *Nature Medicine,* 1999, vol. 5 (7), 780-7
- **GLADUE RP et al.** *Cancer Immunol Immunother,* 2011, vol. 60 (7), 1009-17
- **VONDERHEIDE RH.** *Clin CancerRes,* 2007, vol. 13, 1083-8
- **STAMENKOVIC et al.** *EMBO J.,* 1989, vol. 8, 1403-10
- pharmaceutically acceptable carriers. Remington's Pharmaceutical Sciences 16th edition. 1980
- **KABAT et al.** Sequences of Proteins of Immunological Interest. Public Health Service, National Institutes of Health, 1991
- **BOACKLE, R.J. et al.** *Nature,* 1979, vol. 282, 742-743
- **LUKAS, T.J. et al.** *J. Immunol.,* 1981, vol. 127, 2555-2560
- **BRUNHOUSE, R. ; CEBRA, J.J.** *Mol. Immunol.,* 1979, vol. 16, 907-917
- **BURTON, D.R. et al.** *Nature,* 1980, vol. 288, 338-344
- **THOMMESEN, J.E. et al.** *Mol. Immunol.,* 2000, vol. 37, 995-1004
- **IDUSOGIE, E.E. et al.** *J. Immunol.,* 2000, vol. 164, 4178-4184
- **HEZAREH, M. et al.** *J. Virology,* 2001, vol. 75, 12161-12168
- **MORGAN, A. et al.** *Immunology,* 1995, vol. 86, 319-324
- **JOHNSON, G. ; WU, T.T.** *Nucleic Acids Res.,* 2000, vol. 28, 214-218
- **MAKRIDES, S.C.** *Protein Expr. Purif.,* 1999, vol. 17, 183-202
- **GEISSE, S. et al.** *Protein Expr. Purif.,* 1996, vol. 8, 271-282
- **KAUFMAN, R.J.** *Mol. Biotechnol.,* 2000, vol. 16, 151-161
- **WERNER, R.G.** *Drug Res.,* 1998, vol. 48, 870-880
- Current Protocols in Molecular Biology. Publishing and Wiley Interscience, 1987
- **BARNES, L.M. et al.** *Cytotechnology,* 2000, vol. 32, 109-123
- **BARNES, L.M. et al.** *Biotech. Bioeng.,* 2001, vol. 73, 261-270
- **DUROCHER, Y. et al.** *Nucl. Acids. Res.,* 2002, vol. 30, E9
- **ORLANDI, R. et al.** *Proc. Natl. Acad. Sci. USA,* 1989, vol. 86, 3833-3837
- **CARTER, P. et al.** *Proc. Natl. Acad. Sci. USA,* 1992, vol. 89, 4285-4289
- **NORDERHAUG, L. et al.** *J. Immunol. Methods,* 1997, vol. 204, 77-87
- **SCHLAEGER, E.-J. ; CHRISTENSEN, K.** *Cytotechnology,* 1999, vol. 30, 71-83
- **SCHLAEGER, E.-J.** *J. Immunol. Methods,* 1996, vol. 194, 191-199